# EUROPEAN PATENT SPECIFICATION

(11) **EP 3 359 561 B1**
(45) Date of publication and mention of the grant of the patent: **30.12.2020**
(21) Application number: 16853191.1
(22) Date of filing: 01.12.2016
(51) Int. Cl.: C07K 14/47, C12N 9/64, A61K 38/17, C12N 15/63, C12N 15/81, C12N 5/16

(54) **MODULATORS OF COMPLEMENT FUNCTION**
MODULATOREN EINER KOMPLEMENTFUNKTION
MODULATEURS DE LA FONCTION DU COMPLÉMENT

(30) Priority: 07.10.2016 US 201615288830
(43) Date of publication of application: 15.08.2018
(73) Proprietor: Fritzinger, David C., Monroe, New Jersey 08831 (US); Benjamin, Daniel E., Millstone Township, New Jersey 08535 (US)
(72) Inventor: Fritzinger, David C., Monroe, New Jersey 08831 (US); Benjamin, Daniel E., Millstone Township, New Jersey 08535 (US)
(74) Representative: Loo, Chi Ching
(86) International application number: PCT/IB2016/057272
(87) International publication number: WO 2017/060891

(56) References cited:
- WO-A1-2005/003159
- WO-A2-96/07738
- WO-A2-2005/107785
- US-A1- 2010 179 092
- US-B1- 6 221 657
- US-B1- 6 268 485
- ENRIQUE L LARGHI ET AL: "Modulators of complement activation: a patent review (2008 - 2013)", EXPERT OPINION ON THERAPEUTIC PATENTS, vol. 24, no. 6, 1 June 2014 (2014-06-01), pages 665-686, XP055161526, ISSN: 1354-3776, DOI: 10.1517/13543776.2014.898063
- CARL-WILHELM VOGEL ET AL: "Humanized cobra venom factor: Structure, activity, and therapeutic efficacy in preclinical disease models", MOLECULAR IMMUNOLOGY., vol. 61, no. 2, 1 October 2014 (2014-10-01), pages 191-203, XP055423384, GB ISSN: 0161-5890, DOI: 10.1016/j.molimm.2014.06.035
- MASTELLOS DIMITRIOS C ET AL: "From orphan drugs to adopted therapies: Advancing C3-targeted intervention to the clinical stage", IMMUNOBIOLOGY, URBAN UND FISCHER VERLAG, DE, vol. 221, no. 10, 16 June 2016 (2016-06-16), pages 1046-1057, XP029684330, ISSN: 0171-2985, DOI: 10.1016/J.IMBIO.2016.06.013
- CARL-WILHELM VOGEL ET AL: "Summary", THROMBOSIS AND HAEMOSTASIS, vol. 113, no. 03, 1 May 2015 (2015-05-01), pages 548-552, XP055556312, DE ISSN: 0340-6245, DOI: 10.1160/TH14-04-0300
- CARL-WILHELM VOGEL ET AL: "Cobra venom factor: Structure, function, and humanization for therapeutic complement depletion", TOXICON, vol. 56, no. 7, 1 December 2010 (2010-12-01), pages 1198-1222, XP055046258, ISSN: 0041-0101, DOI: 10.1016/j.toxicon.2010.04.007
- VERONESE FRANCE SCO M. ET AL.: '«Peptide and protein PEGylation: a review of problems and solutions»' BIOMATERIALS vol. 22, no. 5, 2001, pages 405 - 417, XP004227886
- GARCIA BRANDON L. ET AL.: '«Molecular Basis for Complement Recognition and Inhibition Determined by Crystallographic Studies of the Staphylococcal Complement Inhibitor (SCIN) Bound to C3c and C3b»' J MOL BIOL. vol. 402, no. 1, 10 September 2010, pages 17 - 29, XP027244119

### SEQUENCE LISTING

<110> Fritzinger, David C Benjamin, Daniel E
<120> Modulators of Complement Function
<130> TPC32415
<150> US 62/238,512
   <151> 2015-10-07
<150> US 15/288,830
   <151> 2016-10-07
<160> 2
<170> PatentIn version 3.5
<210> 1
   <211> 1642
   <212> PRT
   <213> Homo sapiens
<400> 1 
Gly Ser Pro Met Tyr Ser Ile Ile Thr Pro Asn Ile Leu Arg Leu Glu
1               5                   10                  15

Ser Glu Glu Thr Met Val Leu Glu Ala His Asp Ala Gln Gly Asp Val
            20                  25                  30

Pro Val Thr Val Thr Val His Asp Phe Pro Gly Lys Lys Leu Val Leu
        35                  40                  45

Ser Ser Glu Lys Thr Val Leu Thr Pro Ala Thr Asn His Met Gly Asn
    50                  55                  60

Val Thr Phe Thr Ile Pro Ala Asn Arg Glu Phe Lys Ser Glu Lys Gly
65                  70                  75                  80

Arg Asn Lys Phe Val Thr Val Gln Ala Thr Phe Gly Thr Gln Val Val
                85                  90                  95

Glu Lys Val Val Leu Val Ser Leu Gln Ser Gly Tyr Leu Phe Ile Gln
            100                 105                 110

Thr Asp Lys Thr Ile Tyr Thr Pro Gly Ser Thr Val Leu Tyr Arg Ile
        115                 120                 125

Phe Thr Val Asn His Lys Leu Leu Pro Val Gly Arg Thr Val Met Val
    130                 135                 140

Asn Ile Glu Asn Pro Glu Gly Ile Pro Val Lys Gln Asp Ser Leu Ser
 
145                 150                 155                 160

Ser Gln Asn Gln Leu Gly Val Leu Pro Leu Ser Trp Asp Ile Pro Glu
                165                 170                 175

Leu Val Asn Met Gly Gln Trp Lys Ile Arg Ala Tyr Tyr Glu Asn Ser
            180                 185                 190

Pro Gln Gln Val Phe Ser Thr Glu Phe Glu Val Lys Glu Tyr Val Leu
        195                 200                 205

Pro Ser Phe Glu Val Ile Val Glu Pro Thr Glu Lys Phe Tyr Tyr Ile
    210                 215                 220

Tyr Asn Glu Lys Gly Leu Glu Val Thr Ile Thr Ala Arg Phe Leu Tyr
225                 230                 235                 240

Gly Lys Lys Val Glu Gly Thr Ala Phe Val Ile Phe Gly Ile Gln Asp
                245                 250                 255

Gly Glu Gln Arg Ile Ser Leu Pro Glu Ser Leu Lys Arg Ile Pro Ile
            260                 265                 270

Glu Asp Gly Ser Gly Glu Val Val Leu Ser Arg Lys Val Leu Leu Asp
        275                 280                 285

Gly Val Gln Asn Leu Arg Ala Glu Asp Leu Val Gly Lys Ser Leu Tyr
    290                 295                 300

Val Ser Ala Thr Val Ile Leu His Ser Gly Ser Asp Met Val Gln Ala
305                 310                 315                 320

Glu Arg Ser Gly Ile Pro Ile Val Thr Ser Pro Tyr Gln Ile His Phe
                325                 330                 335

Thr Lys Thr Pro Lys Tyr Phe Lys Pro Gly Met Pro Phe Asp Leu Met
            340                 345                 350

Val Phe Val Thr Asn Pro Asp Gly Ser Pro Ala Tyr Arg Val Pro Val
        355                 360                 365

Ala Val Gln Gly Glu Asp Thr Val Gln Ser Leu Thr Gln Gly Asp Gly
    370                 375                 380

Val Ala Lys Leu Ser Ile Asn Thr His Pro Ser Gln Lys Pro Leu Ser
385                 390                 395                 400
 
Ile Thr Val Arg Thr Lys Lys Gln Glu Leu Ser Glu Ala Glu Gln Ala
                405                 410                 415

Thr Arg Thr Met Gln Ala Leu Pro Tyr Ser Thr Val Gly Asn Ser Asn
            420                 425                 430

Asn Tyr Leu His Leu Ser Val Leu Arg Thr Glu Leu Arg Pro Gly Glu
        435                 440                 445

Thr Leu Asn Val Asn Phe Leu Leu Arg Met Asp Arg Ala His Glu Ala
    450                 455                 460

Lys Ile Arg Tyr Tyr Thr Tyr Leu Ile Met Asn Lys Gly Arg Leu Leu
465                 470                 475                 480

Lys Ala Gly Arg Gln Val Arg Glu Pro Gly Gln Asp Leu Val Val Leu
                485                 490                 495

Pro Leu Ser Ile Thr Thr Asp Phe Ile Pro Ser Phe Arg Leu Val Ala
            500                 505                 510

Tyr Tyr Thr Leu Ile Gly Ala Ser Gly Gln Arg Glu Val Val Ala Asp
        515                 520                 525

Ser Val Trp Val Asp Val Lys Asp Ser Cys Val Gly Ser Leu Val Val
    530                 535                 540

Lys Ser Gly Gln Ser Glu Asp Arg Gln Pro Val Pro Gly Gln Gln Met
545                 550                 555                 560

Thr Leu Lys Ile Glu Gly Asp His Gly Ala Arg Val Val Leu Val Ala
                565                 570                 575

Val Asp Lys Gly Val Phe Val Leu Asn Lys Lys Asn Lys Leu Thr Gln
            580                 585                 590

Ser Lys Ile Trp Asp Val Val Glu Lys Ala Asp Ile Gly Cys Thr Pro
        595                 600                 605

Gly Ser Gly Lys Asp Tyr Ala Gly Val Phe Ser Asp Ala Gly Leu Thr
    610                 615                 620

Phe Thr Ser Ser Ser Gly Gln Gln Thr Ala Gln Arg Ala Glu Leu Gln
625                 630                 635                 640

Cys Pro Gln Pro Ala Ala Arg Arg Arg Arg Ser Val Gln Leu Thr Glu
                645                 650                 655
 
Lys Arg Met Asp Lys Val Gly Lys Tyr Pro Lys Glu Leu Arg Lys Cys
            660                 665                 670

Cys Glu Asp Gly Met Arg Glu Asn Pro Met Arg Phe Ser Cys Gln Arg
        675                 680                 685

Arg Thr Arg Phe Ile Ser Leu Gly Glu Ala Cys Lys Lys Val Phe Leu
    690                 695                 700

Asp Cys Cys Asn Tyr Ile Thr Glu Leu Arg Arg Gln His Ala Arg Ala
705                 710                 715                 720

Ser His Leu Gly Leu Ala Arg Ser Asn Leu Asp Glu Asp Ile Ile Ala
                725                 730                 735

Glu Glu Asn Ile Val Ser Arg Ser Glu Phe Pro Glu Ser Trp Leu Trp
            740                 745                 750

Asn Val Glu Asp Leu Lys Glu Pro Pro Lys Asn Gly Ile Ser Thr Lys
        755                 760                 765

Leu Met Asn Ile Phe Leu Lys Asp Ser Ile Thr Thr Trp Glu Ile Leu
    770                 775                 780

Ala Val Ser Met Ser Asp Lys Lys Gly Ile Cys Val Ala Asp Pro Phe
785                 790                 795                 800

Glu Val Thr Val Met Gln Asp Phe Phe Ile Asp Leu Arg Leu Pro Tyr
                805                 810                 815

Ser Val Val Arg Asn Glu Gln Val Glu Ile Arg Ala Val Leu Tyr Asn
            820                 825                 830

Tyr Arg Gln Asn Gln Glu Leu Lys Val Arg Val Glu Leu Leu His Asn
        835                 840                 845

Pro Ala Phe Cys Ser Leu Ala Thr Thr Lys Arg Arg His Gln Gln Thr
    850                 855                 860

Val Thr Ile Pro Pro Lys Ser Ser Leu Ser Val Pro Tyr Val Ile Val
865                 870                 875                 880

Pro Leu Lys Thr Gly Leu Gln Glu Val Glu Val Lys Ala Ala Val Tyr
                885                 890                 895

His His Phe Ile Ser Asp Gly Val Arg Lys Ser Leu Lys Val Val Pro
            900                 905                 910
 
Glu Gly Ile Arg Met Asn Lys Thr Val Ala Val Arg Thr Leu Asp Pro
        915                 920                 925

Glu Arg Leu Gly Arg Glu Gly Val Gln Lys Glu Asp Ile Pro Pro Ala
    930                 935                 940

Asp Leu Ser Asp Gln Val Pro Asp Thr Glu Ser Glu Thr Arg Ile Leu
945                 950                 955                 960

Leu Gln Gly Thr Pro Val Ala Gln Met Thr Glu Asp Ala Val Asp Ala
                965                 970                 975

Glu Arg Leu Lys His Leu Ile Val Thr Pro Ser Gly Cys Gly Glu Gln
            980                 985                 990

Asn Met Ile Gly Met Thr Pro Thr Val Ile Ala Val His Tyr Leu Asp
        995                 1000                1005

Glu Thr Glu Gln Trp Glu Lys Phe Gly Leu Glu Lys Arg Gln Gly
    1010                1015                1020

Ala Leu Glu Leu Ile Lys Lys Gly Tyr Thr Gln Gln Leu Ala Phe
    1025                1030                1035

Arg Gln Pro Ser Ser Ala Phe Ala Ala Phe Val Lys Arg Ala Pro
    1040                1045                1050

Ser Thr Trp Leu Thr Ala Tyr Val Val Lys Val Phe Ser Leu Ala
    1055                1060                1065

Val Asn Leu Ile Ala Ile Asp Ser Gln Val Leu Cys Gly Ala Val
    1070                1075                1080

Lys Trp Leu Ile Leu Glu Lys Gln Lys Pro Asp Gly Val Phe Gln
    1085                1090                1095

Glu Asp Ala Pro Val Ile His Gln Glu Met Ile Gly Gly Leu Arg
    1100                1105                1110

Asn Asn Asn Glu Lys Asp Met Ala Leu Thr Ala Phe Val Leu Ile
    1115                1120                1125

Ser Leu Gln Glu Ala Lys Asp Ile Cys Glu Glu Gln Val Asn Ser
    1130                1135                1140

Leu Pro Gly Ser Ile Thr Lys Ala Gly Asp Phe Leu Glu Ala Asn
 
1145                1150                1155

Tyr Met Asn Leu Gln Arg Ser Tyr Thr Val Ala Ile Ala Gly Tyr
    1160                1165                1170

Ala Leu Ala Gln Met Gly Arg Leu Lys Gly Pro Leu Leu Asn Lys
    1175                1180                1185

Phe Leu Thr Thr Ala Lys Asp Lys Asn Arg Trp Glu Asp Pro Gly
    1190                1195                1200

Lys Gln Leu Tyr Asn Val Glu Ala Thr Ser Tyr Ala Leu Leu Ala
    1205                1210                1215

Leu Leu Gln Leu Lys Asp Phe Asp Phe Val Pro Pro Val Val Arg
    1220                1225                1230

Trp Leu Asn Glu Gln Arg Tyr Tyr Gly Gly Gly Tyr Gly Ser Thr
    1235                1240                1245

Gln Ala Thr Phe Met Val Phe Gln Ala Leu Ala Gln Tyr Gln Lys
    1250                1255                1260

Asp Ala Pro Asp His Gln Glu Leu Asn Leu Asp Val Ser Leu Gln
    1265                1270                1275

Leu Pro Ser Arg Ser Ser Lys Ile Thr His Arg Ile His Trp Glu
    1280                1285                1290

Ser Ala Ser Leu Leu Arg Ser Glu Glu Thr Lys Glu Asn Glu Gly
    1295                1300                1305

Phe Thr Val Thr Ala Glu Gly Lys Gly Gln Gly Thr Leu Ser Val
    1310                1315                1320

Val Thr Met Tyr His Ala Lys Ala Lys Asp Gln Leu Thr Cys Asn
    1325                1330                1335

Lys Phe Asp Leu Lys Val Thr Ile Lys Pro Ala Pro Glu Thr Glu
    1340                1345                1350

Lys Arg Pro Gln Asp Ala Lys Asn Thr Met Ile Leu Glu Ile Cys
    1355                1360                1365

Thr Arg Tyr Arg Gly Asp Gln Asp Ala Thr Met Ser Ile Leu Asp
    1370                1375                1380
 
Ile Ser Met Met Thr Gly Phe Ala Pro Asp Thr Asp Asp Leu Lys
    1385                1390                1395

Gln Leu Ala Asn Gly Val Asp Arg Tyr Ile Ser Lys Tyr Glu Leu
    1400                1405                1410

Asp Lys Ala Phe Ser Asp Arg Asn Thr Leu Ile Ile Tyr Leu Asp
    1415                1420                1425

Lys Val Ser His Ser Glu Asp Asp Cys Leu Ala Phe Lys Val His
    1430                1435                1440

Gln Tyr Phe Asn Val Glu Leu Ile Gln Pro Gly Ala Val Lys Val
    1445                1450                1455

Tyr Ala Tyr Tyr Asn Leu Glu Glu Ser Cys Thr Arg Phe Tyr His
    1460                1465                1470

Pro Glu Lys Glu Asp Gly Lys Leu Asn Lys Leu Cys Arg Asp Glu
    1475                1480                1485

Leu Cys Arg Cys Ala Glu Glu Asn Cys Phe Ile Gln Lys Ser Asp
    1490                1495                1500

Asp Lys Val Thr Leu Glu Glu Arg Leu Asp Lys Ala Cys Glu Pro
    1505                1510                1515

Gly Val Asp Tyr Val Tyr Lys Thr Arg Leu Val Lys Val Gln Leu
    1520                1525                1530

Ser Asn Asp Phe Asp Glu Tyr Ile Met Ala Ile Glu Gln Thr Ile
    1535                1540                1545

Lys Ser Gly Ser Asp Glu Val Gln Val Gly Gln Gln Arg Thr Phe
    1550                1555                1560

Ile Ser Pro Ile Lys Cys Arg Glu Ala Leu Lys Leu Glu Glu Lys
    1565                1570                1575

Lys His Tyr Leu Met Trp Gly Leu Ser Ser Asp Phe Trp Gly Glu
    1580                1585                1590

Lys Pro Asn Leu Ser Tyr Ile Ile Gly Lys Asp Thr Trp Val Glu
    1595                1600                1605

His Trp Pro Glu Glu Asp Glu Cys Gln Asp Glu Glu Asn Gln Lys
    1610                1615                1620
 
Gln Cys Gln Asp Leu Gly Ala Phe Thr Glu Ser Met Val Val Phe
    1625                1630                1635

Gly Cys Pro Asn
    1640
<210> 2
   <211> 4926
   <212> DNA
   <213> Homo sapiens
<400> 2

agtcccatgt actctatcat cacccccaac atcttgcggc tggagagcga ggagaccatg 60

gtgctggagg cccacgacgc gcaaggggat gttccagtca ctgttactgt ccacgacttc 120

ccaggcaaaa aactagtgct gtccagtgag aagactgtgc tgacccctgc caccaaccac 180

atgggcaacg tcaccttcac gatcccagcc aacagggagt tcaagtcaga aaaggggcgc 240

aacaagttcg tgaccgtgca ggccaccttc gggacccaag tggtggagaa ggtggtgctg 300

gtcagcctgc agagcgggta cctcttcatc cagacagaca agaccatcta cacccctggc 360

tccacagttc tctatcggat cttcaccgtc aaccacaagc tgctacccgt gggccggacg 420

gtcatggtca acattgagaa cccggaaggc atcccggtca agcaggactc cttgtcttct 480

cagaaccagc ttggcgtctt gcccttgtct tgggacattc cggaactcgt caacatgggc 540

cagtggaaga tccgagccta ctatgaaaac tcaccacagc aggtcttctc cactgagttt 600

gaggtgaagg agtacgtgct gcccagtttc gaggtcatag tggagcctac agagaaattc 660

tactacatct ataacgagaa gggcctggag gtcaccatca ccgccaggtt cctctacggg 720

aagaaagtgg agggaactgc ctttgtcatc ttcgggatcc aggatggcga acagaggatt 780

tccctgcctg aatccctcaa gcgcattccg attgaggatg gctcggggga ggttgtgctg 840

agccggaagg tactgctgga cggggtgcag aacctccgag cagaagacct ggtggggaag 900

tctttgtacg tgtctgccac cgtcatcttg cactcaggca gtgacatggt gcaggcagag 960

cgcagcggga tccccatcgt gacctctccc taccagatcc acttcaccaa gacacccaag 1020

tacttcaaac caggaatgcc ctttgacctc atggtgttcg tgacgaaccc tgatggctct 1080

ccagcctacc gagtccccgt ggcagtccag ggcgaggaca ctgtgcagtc tctaacccag 1140

ggagatggcg tggccaaact cagcatcaac acacacccca gccagaagcc cttgagcatc 1200

acggtgcgca cgaagaagca ggagctctcg gaggcagagc aggctaccag gaccatgcag 1260

gctctgccct acagcaccgt gggcaactcc aacaattacc tgcatctctc agtgctacgt 1320

acagagctca gacccgggga gaccctcaac gtcaacttcc tcctgcgaat ggaccgcgcc 1380

cacgaggcca agatccgcta ctacacctac ctgatcatga acaagggcag gctgttgaag 1440

gcgggacgcc aggtgcgaga gcccggccag gacctggtgg tgctgcccct gtccatcacc 1500

accgacttca tcccttcctt ccgcctggtg gcgtactaca cgctgatcgg tgccagcggc 1560

cagagggagg tggtggccga ctccgtgtgg gtggacgtca aggactcctg cgtgggctcg 1620

ctggtggtaa aaagcggcca gtcagaagac cggcagcctg tacctgggca gcagatgacc 1680

ctgaagatag agggtgacca cggggcccgg gtggtactgg tggccgtgga caagggcgtg 1740

ttcgtgctga ataagaagaa caaactgacg cagagtaaga tctgggacgt ggtggagaag 1800

gcagacatcg gctgcacccc gggcagtggg aaggattacg ccggtgtctt ctccgacgca 1860

gggctgacct tcacgagcag cagtggccag cagaccgccc agagggcaga acttcagtgc 1920

ccgcagccag ccgcccgccg acgccgttcc gtgcagctca cggagaagcg aatggacaaa 1980

gtcggcaagt accccaagga gctgcgcaag tgctgcgagg acggcatgcg ggagaacccc 2040

atgaggttct cgtgccagcg ccggacccgt ttcatctccc tgggcgaggc gtgcaagaag 2100

gtcttcctgg actgctgcaa ctacatcaca gagctgcggc ggcagcacgc gcgggccagc 2160

cacctgggcc tggccaggag taacctggat gaggacatca ttgcagaaga gaacatcgtt 2220

tcccgaagtg agttcccaga gagctggctg tggaacgttg aggacttgaa agagccaccg 2280

aaaaatggaa tctctacgaa gctcatgaat atatttttga aagactccat caccacgtgg 2340

gagattctgg ctgtcagcat gtcggacaag aaagggatct gtgtggcaga ccccttcgag 2400

gtcacagtaa tgcaggactt cttcatcgac ctgcggctac cctactctgt tgttcgaaac 2460

gagcaggtgg aaatccgagc cgttctctac aattaccggc agaaccaaga gctcaaggtg 2520

agggtggaac tactccacaa tccagccttc tgcagcctgg ccaccaccaa gaggcgtcac 2580

cagcagaccg taaccatccc ccccaagtcc tcgttgtccg ttccatatgt catcgtgccg 2640

ctaaagaccg gcctgcagga agtggaagtc aaggctgccg tctaccatca tttcatcagt 2700

gacggtgtca ggaagtccct gaaggtcgtg ccggaaggaa tcagaatgaa caaaactgtg 2760

gctgttcgca ccctggatcc agaacgcctg ggccgtgaag gagtgcagaa agaggacatc 2820

ccacctgcag acctcagtga ccaagtcccg gacaccgagt ctgagaccag aattctcctg 2880

caagggaccc cagtggccca gatgacagag gatgccgtcg acgcggaacg gctgaagcac 2940

ctcattgtga ccccctcggg ctgcggggaa cagaacatga tcggcatgac gcccacggtc 3000

atcgctgtgc attacctgga tgaaacggag cagtgggaga agttcggcct agagaagcgg 3060

cagggggcct tggagctcat caagaagggg tacacccagc agctggcctt cagacaaccc 3120

agctctgcct ttgcggcctt cgtgaaacgg gcacccagca cctggctgac cgcctacgtg 3180

gtcaaggtct tctctctggc tgtcaacctc atcgccatcg actcccaagt cctctgcggg 3240

gctgttaaat ggctgatcct ggagaagcag aagcccgacg gggtcttcca ggaggatgcg 3300

cccgtgatac accaagaaat gattggtgga ttacggaaca acaacgagaa agacatggcc 3360

ctcacggcct ttgttctcat ctcgctgcag gaggctaaag atatttgcga ggagcaggtc 3420

aacagcctgc caggcagcat cactaaagca ggagacttcc ttgaagccaa ctacatgaac 3480

ctacagagat cctacactgt ggccattgct ggctatgctc tggcccagat gggcaggctg 3540

aaggggcctc ttcttaacaa atttctgacc acagccaaag ataagaaccg ctgggaggac 3600

cctggtaagc agctctacaa cgtggaggcc acatcctatg ccctcttggc cctactgcag 3660

ctaaaagact ttgactttgt gcctcccgtc gtgcgttggc tcaatgaaca gagatactac 3720

ggtggtggct atggctctac ccaggccacc ttcatggtgt tccaagcctt ggctcaatac 3780

caaaaggacg cccctgacca ccaggaactg aaccttgatg tgtccctcca actgcccagc 3840

cgcagctcca agatcaccca ccgtatccac tgggaatctg ccagcctcct gcgatcagaa 3900

gagaccaagg aaaatgaggg tttcacagtc acagctgaag gaaaaggcca aggcaccttg 3960

tcggtggtga caatgtacca tgctaaggcc aaagatcaac tcacctgtaa taaattcgac 4020

ctcaaggtca ccataaaacc agcaccggaa acagaaaaga ggcctcagga tgccaagaac 4080

actatgatcc ttgagatctg taccaggtac cggggagacc aggatgccac tatgtctata 4140

ttggacatat ccatgatgac tggctttgct ccagacacag atgacctgaa gcagctggcc 4200

aatggtgttg acagatacat ctccaagtat gagctggaca aagccttctc cgataggaac 4260

accctcatca tctacctgga caaggtctca cactctgagg atgactgtct agctttcaaa 4320

gttcaccaat actttaatgt agagcttatc cagcctggag cagtcaaggt ctacgcctat 4380

tacaacctgg aggaaagctg tacccggttc taccatccgg aaaaggagga tggaaagctg 4440

aacaagctct gccgtgatga actgtgccgc tgtgctgagg agaattgctt catacaaaag 4500

tcggatgaca aggtcaccct ggaagaacgg ctggacaagg cctgtgagcc aggagtggac 4560

tatgtgtaca agacccgact ggtcaaggtt cagctgtcca atgactttga cgagtacatc 4620

atggccattg agcagaccat caagtcaggc tcggatgagg tgcaggttgg acagcagcgc 4680

acgttcatca gccccatcaa gtgcagagaa gccctgaagc tggaggagaa gaaacactac 4740

ctcatgtggg gtctctcctc cgatttctgg ggagagaagc ccaacctcag ctacatcatc 4800

gggaaggaca cttgggtgga gcactggcct gaggaggacg aatgccaaga cgaagagaac 4860

cagaaacaat gccaggacct cggcgccttc accgagagca tggttgtctt tgggtgcccc 4920

aactga 4926

## Description

### FIELD OF THE INVENTION

The invention relates generally to a modified human C3 protein containing a number of single amino acid changes in the α- and β-chain of human C3 protein, designed to increase the affinity of the modified protein to factor B or its active peptide Bb, to decrease the affinity of the modified protein to factor H, and to reduce the immunogenicity of the modified protein as compared to native human C3 protein; a nucleotide sequence encoding the modified C3 protein; a plasmid or viral vector containing the nucleotide sequence for expression the modified C3 protein; and a host cell containing the plasmid or viral vector. The invention also relates to a polyethylene glycol covalently bound to the modified C3 protein for reducing immunogenicity and increasing plasma half-life of the modified C3 protein; a method for depleting complement in a patient by administering the modified C3 protein to the patient in an amount effective to deplete complement; and a method of ameliorating effects caused by certain diseases or a method of ameliorating reperfusion injury in a patient by delivering an effective amount of the modified C3 protein sufficient to deplete complement.

### BACKGROUND OF THE INVENTION

The third component of complement, C3, plays an extremely important role in all three of the complement activation pathways, the classical, alternative and lectin pathways **(1, 2, 3).** In addition, many of the C3 activation products have important functions in immune response and host defense **(4, 5).** In all the activation pathways, activated C3, called C3b, is a structural subunit of the C3 convertase. The convertase is formed when C3b binds to another complement protein, factor B, which is cleaved by another complement protein, factor D. This yields the activation peptides of factor B, Ba, and Bb, where Bb binds to C3b. This C3 convertase C3b,Bb, is then able to activate more C3 molecules in a similar manner. This cleavage also breaks a high-energy thioester linkage, allowing the covalent attachment of C3b to nearby cell surfaces **(6).** As more C3 molecules are activated, a tri- molecular complex C3b2,Bb (the C5 convertase), will be formed, which is able to activate C5.

There is a C3 analogue found in cobra venom, called cobra venom factor (CVF) that is a structurally and functionally similar to C3b **(7).** Like C3b, CVF is able to bind factor B, which is cleaved by factor D to yield a C3/C5 convertase, CVF,Bb. Similar to C3b,Bb, CVF,Bb is able to cleave C3 molecules in an identical manner. However, the CVF-containing convertase is intrinsically far more stable than the C3b,Bb convertase **(8).** It is also resistant to regulation by a number of complement regulatory proteins. Finally, unlike C3b,Bb, CVF,Bb acts in the fluid phase rather than on cell surfaces, and is able to activate C5 without binding an additional C3b.

CVF and C3 have also been shown to be quite similar structurally. This similarity is reflected in protein sequence similarity, electron microscopic ultrastructure, and most importantly, three-dimensional structure as determined by x-ray crystallography **(8, 9).** However, there are differences. C3 is a 2-chain molecule with a molecular mass of about 180 kDaltons, while CVF has a 3-chain structure, with a mass of about 149 kDaltons, resembling the C3c, one of the breakdown products of C3b **(8, 10).**

Because the complement system is involved in a number of different diseases, including some that are wide spread **(4, 5),** there has been a great deal of research in drugs that inhibit complement activation. CVF, and CVF-like proteins are unique in that they are able to deplete complement through exhaustive complement activation **(11).** Using a C3-like protein with CVF-like properties and low immunogenicity would be a novel means of stopping complement activation **(12).** WO 96/07738 discloses native complement pathway proteins modified such that the protein is capable of forming a stable C3 convertase, where the modified protein is preferably a modified human C3 protein, and DNA sequences encoding such proteins are also provided, together with DNA constructs. WO 2005/003159 A1 discloses isolated polypeptides having complement modulating activity. Specifically, the document provides that the isolated polypeptides having complement-depleting properties, i.e. that effect an efficient consumption of complement in human serum, and that human C3- derivatives which are capable of forming C3-convertases exerting an extended CVF, Bb-like half-life of up several hours, compared to 1.5 minutes of the naturally occurring C3-convertases.

### SUMMARY OF THE INVENTION

The following listing of embodiments is a non-limiting statement of various aspects of the invention. Other aspects and variations will be evident in light of the entire disclosure.

A first embodiment includes a human C3 protein (SEQ ID NO 1) or an active fragment thereof modified to form a stable C3 convertase, with the replacement of one amino acid at position 1518 selected from the group consisting of N, Q, H, S, T, or b-hydroxy-norvaline, such that the modified C3 protein forms a more stable complex with factor B and its activated form, Bb.

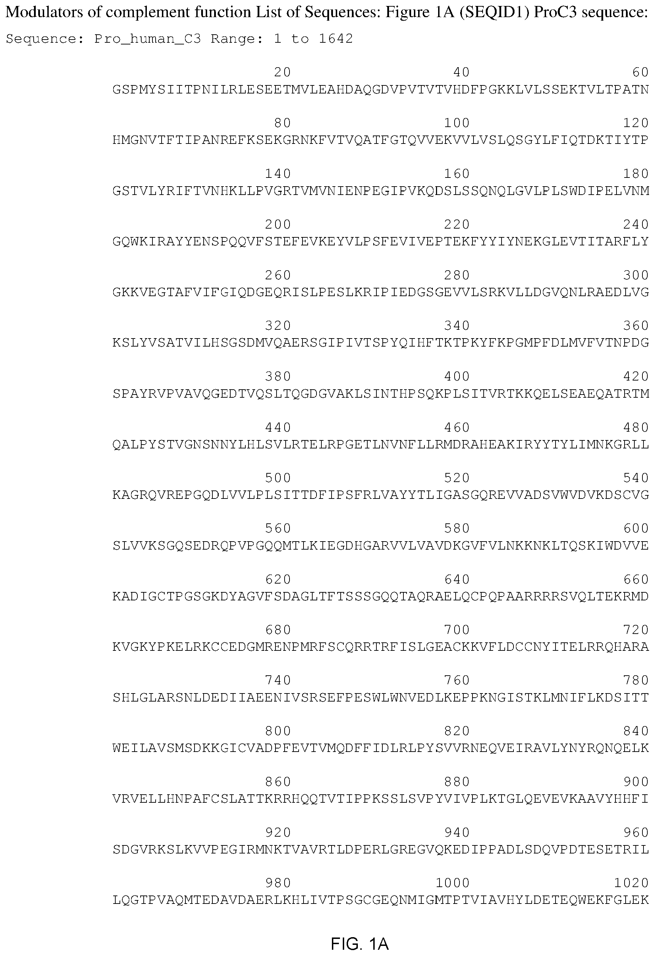

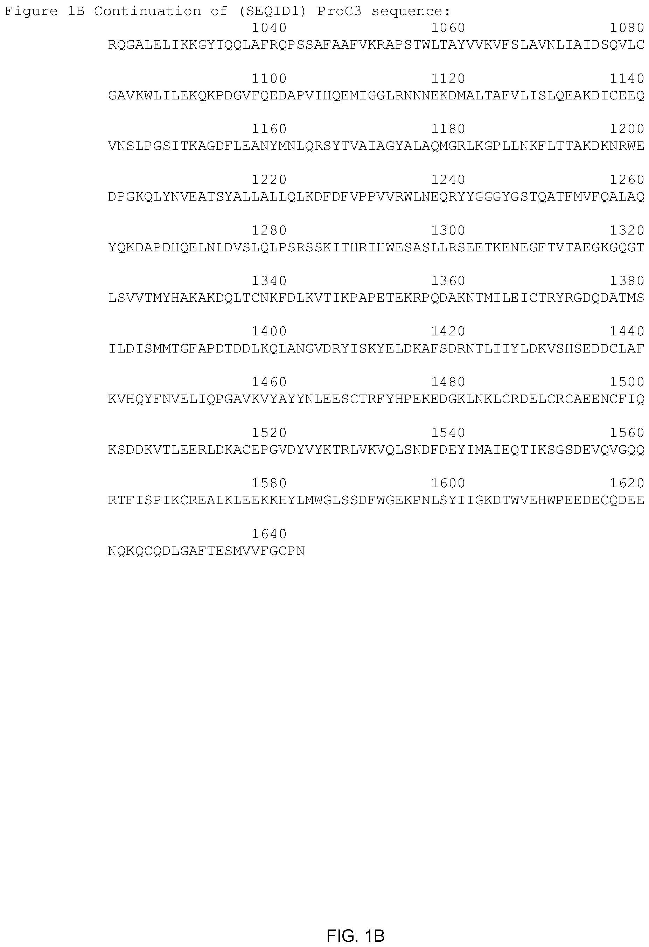

Some embodiments, not in accordance with the present invention, include the replacement of one or more individual amino acids between amino acids 1490 and 1642 (proC3 numbering, **Figure 1****,** SEQ ID NO:1) of human C3, such that the modified C3 protein forms a more stable complex with factor B and its activated form, Bb. Some embodiments, not in accordance with the present invention, include the replacement of one or more individual amino acids between positions 730 and 1350, such that the modified C3 has less affinity for factor H short consensus repeats (SCR), 1-4 and SCRs 19-20 **(13, 14).** Some embodiments, not in accordance with the present invention, contain amino acid substitutions between positions 1 and 1642 such that the resulting protein shows less immunogenicity than native C3. Some embodiments, not in accordance with the present invention, will have the amino acid substitutions designed to increase affinity for Bb while decreasing the affinity for factor H. Some embodiments, not in accordance with the present invention, will have amino acid substitutions designed to increase the affinity of the modified C3 protein for Bb while decreasing the immunogenicity of the protein. Some embodiments, not in accordance with the present invention, will have amino acid substitutions designed to decrease affinity for factor H while decreasing the immunogenicity of the modified C3 protein. Some embodiments, not in accordance with the present invention, will have amino acid substitutions designed to increase the affinity of the modified C3 protein for Bb, decrease the affinity for factor H and decrease the immunogenicity of the resulting protein. In some embodiments, not in accordance with the present invention, the modified protein will cleave C3 but not C5.

In some embodiments, the modified C3 protein can be expressed as a single chain protein. In some embodiments, the modified C3 protein can be cleaved into at least two chains in a form that resembles C3. In further embodiments, the modified C3 protein can be cleaved to release a C3a portion therefrom. The entire C3a sequence (SEQ ID NO:3, amino acid residues 651 through 727 of SEQ ID NO:1, SVQLTEKRMDKVGKYPKELRKCCEDGMRENPMRFSCQRRTRFISLGEAC KKVFLDCCNYITELRRQHARASHLGLAR) can be removed, such that the modified protein has the same structure as C3b. In some embodiments, the amino acids involved in the thioester linkage can be changed, such that the thioester linkage is unable to form, wherein C988 is substituted by one of the amino acids: S, T, Q, or N.

In some embodiments, the modified protein can have an additional 1 to 19 amino acids at the N-terminus that are not encoded by C3 nucleic acids. In some embodiments, the modified protein can include a non-C3 signal peptide, such as a *Drosophila* Bip signal sequence. In some embodiments, not in accordance with the present invention, the modified C3 protein can have modified affinity for factor B and/or factor D. In some embodiments, not in accordance with the present invention, the modified protein can show partial or complete resistance to Factor H and/or Factor I. In some embodiments, the modified protein can be essentially non-immunogenic.

Other embodiments can include a modified C3 protein as claimed in the first embodiment in an effective amount for use in depleting complement in a patient. In some embodiments, the administration can be local. In further embodiments, the local administration can be into an organ, subcutaneously, into a cavity, or into a tissue. In other embodiments, the local administration can employ a targeting function capable of concentrating the modified C3 protein in a desired location. In further embodiments, not in accordance with the present invention, the targeting function can include using an antibody conjugated to the modified C3 protein. In some embodiments, the administration can be a systemic administration, such as intravenous or intraperitoneal.

Further embodiments can be a modified C3 protein as claimed in the first embodiment in an effective amount for use in avoiding or ameliorating reperfusion injury in a patient by depleting complement and permitting reperfusion in the patient. Some embodiments can be a modified C3 protein as claimed in the first embodiment in an effective amount for use in ameliorating effects caused by a disease by depleting complement, wherein the disease is selected from the group consisting of: Neuromyelitis Optica, Multiple Sclerosis, Myasthenia Gravis, Rheumatoid arthritis, wet and dry Age-Related Macular Degeneration, Hemoglobin Urinary Syndrome, Paroxysmal Nocturnal Hemoglobinuria, Inflammatory Bowel Disease, Crohn's Disease, Gout, Alzheimer's disease, Parkinson's Disease, Amyotrophic Lateral Sclerosis, Familial Mediterranean Fever, Dengue Fever, Injury, Dense Deposit Disease, C3 Glomerulonephritis, Neuropathic Pain, and Inflammatory Pain. In some embodiments, the delivering step can include injecting the modified C3 protein into an artery. In other embodiments, the delivering step can include a local delivery of the modified C3 protein. In other embodiments, the delivering step can include a systemic delivery of the modified C3 protein. In some embodiments, reperfusion can be opening a blocked artery. In some embodiments, the reperfusion can occur in connection with transplantation of an organ. In some embodiments, the modified protein could also have polyethylene glycol covalently bound to the n-terminus, the c-terminus, any, some, or all lysines. The bound polyethylene glycol would increase the half-life and stability in the blood following parenteral injection, while decreasing immunogenicity. Not in accordance with the present invention, polyethylene glycol could also be bound to any, some, or all of the other amino acids of the protein.

Other embodiments, not in accordance with the present invention, include methods of selecting a modified C3 protein, by characterizing at least one property of the modified C3 protein to form a function profile of the modified protein; and matching the function profile with a disease or condition to be treated. In some embodiments, at least one property can be selected from the group consisting of: convertase activity, convertase formation, convertase stability, ability to cleave C3, and ability to cleave C5. In some embodiments, the selected C3 protein participates in formation of a convertase adapted for treatment of a chronic condition. In some embodiments, the adaptation can be any of the following: longer plasma half-life, and greater stability. In some embodiments, the convertase can be adapted for treatment of a reperfusion injury. In other embodiments, not in accordance with the present invention, the adaptation can be any of, high convertase activity, resistance to Factor H, and resistance to Factor I.

Some embodiments, not in accordance with the present invention, include a nucleic acid sequence encoding a modified C3 protein, and/or a vector including the nucleic acid and/or a host cell containing the vector. In some embodiments, the host cell can be any of the following: a *Drosophila* S2 cell, an Sf9 cell, a CHO cell, a COS-7 cell, a High Five™cell, a yeast cell, a BHK cell, and an HEK293 cell. Some embodiments include a composition that can include the modified human complement C3 protein and a pharmaceutically acceptable carrier and/or the nucleic acid encoding the modified C3 protein. Some embodiments include an expression system expressing the modified C3 protein. In some embodiment, the expression system include a cell selected from the group consisting of: a *Drosophila* S2 cell, an Sf9 cell, a CHO cell, a COS-7 cell, a High Five™ cell, a yeast cell, a BHK cell, and an HEK293 cell.

### BRIEF DESCRIPTION OF THE DRAWINGS

**Figure 1A** describes the amino acid sequence of human ProC3 (SEQ ID NO:1).
**Figure 1B** is a continuation of the amino acid sequence of human ProC3 (SEQ ID NO: 1).
**Figure 2A** describes the nucleotide sequence of human ProC3 (SEQ ID NO:2).
**Figure 2B** is a continuation of the nucleotide sequence of human ProC3 (SEQ ID NO:2).
**Figure 2C** is a continuation of the nucleotide sequence of human ProC3 (SEQ ID NO:2).
**Figure 2D** is a continuation of the nucleotide sequence of human ProC3 (SEQ ID NO:2).
**Figure 2E** is a continuation of the nucleotide sequence of human ProC3 (SEQ ID NO:2).

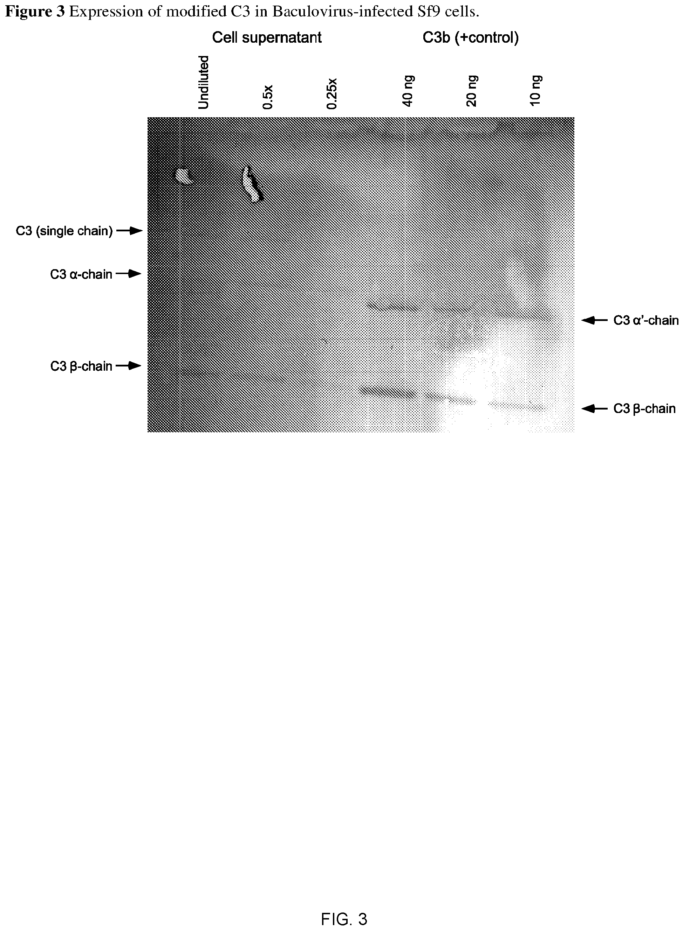

**Figure 3** shows expression of modified C3 in Baculovirus-infected Sf9 cells. Western blot showing expression of sC3 in the Baculovirus insect expression system. Different amounts of supernatant from HighFive cells infected with recombinant Baculovirus are shown on the left three lanes. As a positive control, purified C3b was run on the three lanes on the right. C3b consists of 2 chains, the α'-chain and the β-chain. There are three bands immune-reacting bands in the expressed protein, the β-chain, the α-chain, and protein that has not been processed (pro-sC3). The α'-chain is the α-chain from which the 77 amino acid C3a has been removed. The presence of the single-chain form of C3 shows that the protease responsible for the maturation of pro-C3 to C3 is only present in the Baculovirus supernatants in very limited amounts.

### DETAIL DESCRIPTIONS OF THE INVENTION

Previous studies on human C3/CVF hybrid proteins have shown it is possible to prepare C3 proteins with substitutions of CVF sequences that have many of the properties of CVF, including formation of a stable convertase, reduced factor H affinity, and low immunogenicity **(12).** Combining these studies with the available crystal structures of C3b, CVF, C3b complexed with factor Bb, CVF in a complex with factor B, C3b bound to factor H (SCRs 1 to 4), and C3b bound to factor H (SCRs 19 and 20) **(8, 13, 14)** has suggested which individual amino acid substitutions may allow the formation of more stable convertases and which amino acid substitutions may reduce the affinity of C3b for factor H, thereby increasing the stability of modified C3 proteins in vivo. Additionally, computer programs online, such as the Immune Epitope Database from National Institute of Allergy and Infectious Disease (www.iedb.org/home_v3.php) are able to predict which amino acid sequences within a protein increase the immunogenicity of the protein.

### Example 1: Production of modified human C3 proteins

There are numerous amino acid substitutions in human C3 that should produce a modified human C3 that would have a higher affinity for factor B, thus forming a more stable convertase, a lower affinity for factor H, thus increasing the stability of the modified C3 *in vivo,* and will decrease the immunogenicity of the modified C3 protein.

It has been well documented in the complement literature that Cobra Venom Factor (CVF) forms a more physiochemically stable C3 convertase (CVF,Bb) than does human C3b **(6),** with a half-life of dissociation of 7 hours at 37°C, versus 1.5 minutes for the C3b-containing convertase. Crystal structures have been derived for CVF,B, and C3b,Bb, and interactions between CVF or C3 and factor B (or Bb, respectively) have been determined from the crystal structures. A comparison of the ionic and non-ionic interactions were used to identify the amino acid residues of the C3 protein that interact with factor B or Bb, in which possible amino acid substitutions would result in a more stable C3 convertase. What follows is a list of some of the mutations: P1518N, Q, H, S, T, or β-bydroxy-norvaline; (the following mutations are not in accordance with the present invention) S1550N, D, E, Q, V, I, L, or L-Glu-γ-hydrazide; V1637N, T, S, E, Q, D, or mono-4-fluoroglutamic Acid; N1642I, E, D, Q, T, S, or β-bydroxy-norvaline; G1519I, T, S, V, L, A, N, Q, H, D, E, or L-Glu-γ-hydrazide; A1543Q, S, T, V, L, D, E, N, I, or L*-threo-*β*-* hydroxyl-aspartic acid; I1544G, K, W, V, 3-fluoro-valine, L-*t*-butyl-glycine, L- threonine, or L-*allo*-threonine; E1545D, N, Q, S, T, 5,5',5'-trifluoro-leucine, or β-*t*-butyl- alanine; Q1546S, T, M, A, D, E, N, mono-4-fluoro-glutamic acid, or 4,4-difluoro-glutamic acid; T1547A, V, L, I, G, W, 3-fluoro-valine, L-*t*-butyl-glycine; V1555T, S, D, N, Q, E, or L-Glu-γ-hydrazide; Q1556L, I, M, V, G, P, thiazolidine-2-carboxylic acid, thiazolidine-4-carboxylic acid, 3,4-dehydro-proline, or L-azetidine-2-carboxylic acid; V1557S, T, N, Q, D, E, R, H, K, or L-canavanine; Q1559L, R, H, K, N, Q, *S*-2-aminoethylcysteine, or dehydrolysine; E1633L, M, I, Y, W, F, 3-fluoro-L-tyrosine, or 3-nitro-L-tyrosine; V1636T, S, D, E, or β-hydroxynorvaline; A1630Q, N, T, S, D, E, or L-Glu-γ-hydrazide.

The crystal structures of factor H (CCPs 1-4 or CCPs 19-20) complexed with C3b have been derived **(13, 14).** The structures were used to map the ionic and non-ionic interactions between the two proteins. These interactions suggested amino acid substitutions on C3b that may reduce the strength of the interactions between the two proteins, either with substitutions that will eliminate ionic interactions, or that change the character of amino acids involved in non-ionic interactions between the two proteins by replacing polar residues with non-polar residues, or by making substitutions where a bulky residue is replaced by a small one (e.g. a tryptophane with an alanine or glycine). What follows is a list of the mutations, not in accordance with the present invention: D733G, A, or V; I734F, W, or Y; E738S, or T; N739D, E, L, I, or V; H897D, E, T, S, G, A, V, L, or I; H898A, G, V, D, E, I, or L; K1030T, S, D, E, M, L, or I; T1033G, A, V, L, or I; V1049T, N, Q, D, or E; Q1140W, Y, F, M, S, T, D, or E; T1287R, K, H, N, or Q; H1291F, W, Y, I, L, S, T, D, or E; K1285P, V, A, G, F, D, or E; L1298G, A, or V.

Potentially immunogenic regions of human C3 were determined using available internet programs, and substitutions designed by using similar amino acid residues that are less immunogenic but similar enough to the original residue to prevent loss of function. E176D, S, T, or L; V178A, I, L, M, or G; Q182N, S, T, I, or V; W183F, Y, M, or L; K184H, R, or Y; Y1173F, M, L, or I; A1174G, or V; Q1177N, S, or T; M1178L, I, or V; R1180H, K, T, S, Q, or N; K1182H, R, N, Q, T, or S; K1194R, Q, or N; N1197Q, S, or T; W1199F, Y, M, L, or I; K1204R, H, N, or Q; Y1207L, I, M, or V; V1232A, or G; R1233H, K, N, or Q; W1234F, Y, M, or L; E1237D; Q1238N, T, or S; R1239H, K, N, or Q; Y1240F, M, L, or I; W781F, Y, M, L, or V; I783L, M, V, or A; L784I, M, or V; M788L, I, V, or A; K791A, L, N, or Q; Y5F, W, L, I, or M; I7L, V, or A; S17T, N, or D; R14H, K, N, or Q; E1210N, S, or T; Y1214F, W, I, L, M, or V; L1216I, M, or A; Q1221N, T, or S; K1223R, H, N, or Q; F1252Y, W, L, I, or V; M1253L, I, or V; F1255Y, W, L, I, or V; Q1256N, or T; Y1261W, F, M, I, or L; K115H, R, Q, or N; I117L, V, or A; Y118F, W, L, M, or I; Y513F, W, L, M, or I; Y514F, W L, M, or I; L516I, V, or A; I517L, V, or A; S520T, D, or E.

Stabilization of the protein could also be increased by the attachment of polyethylene glycol on the modified C3 protein.

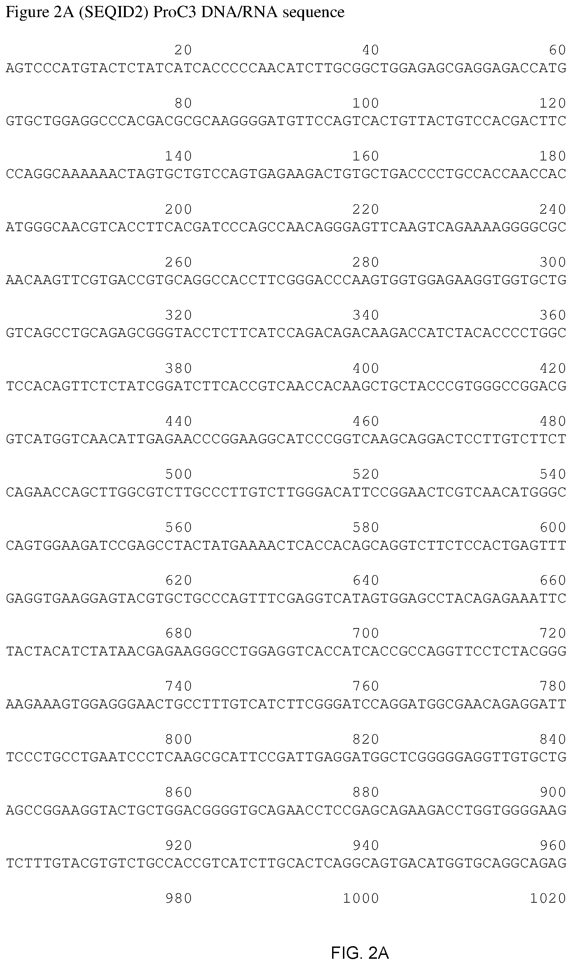

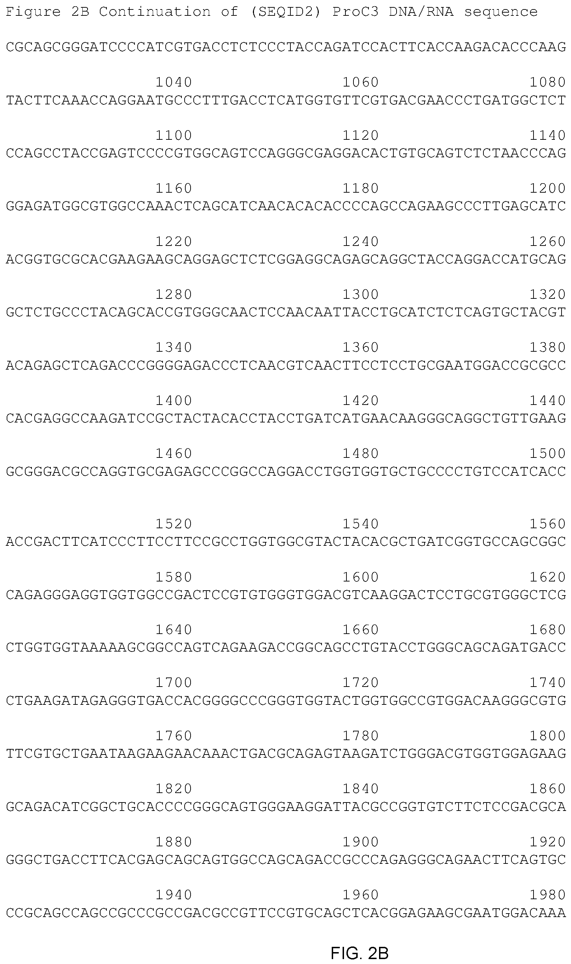

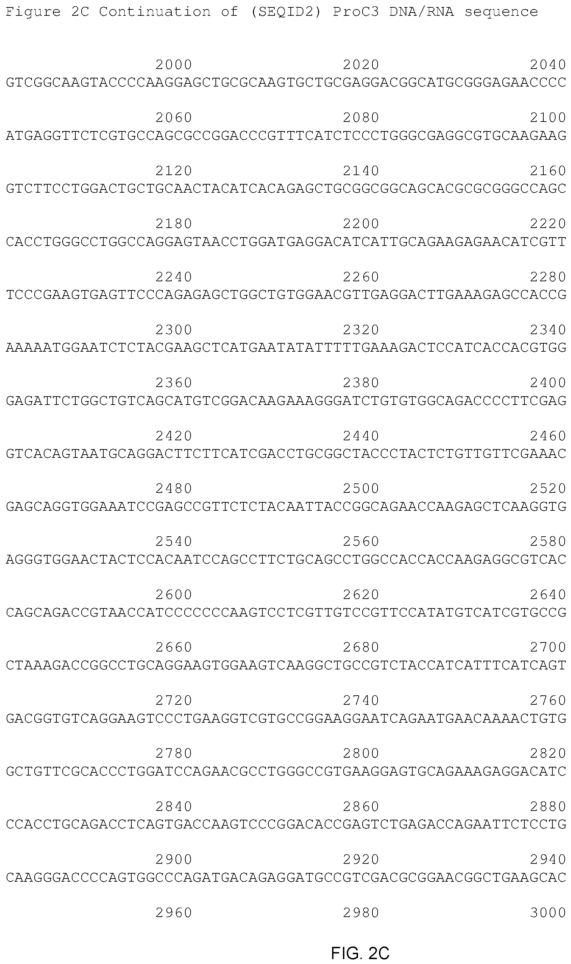

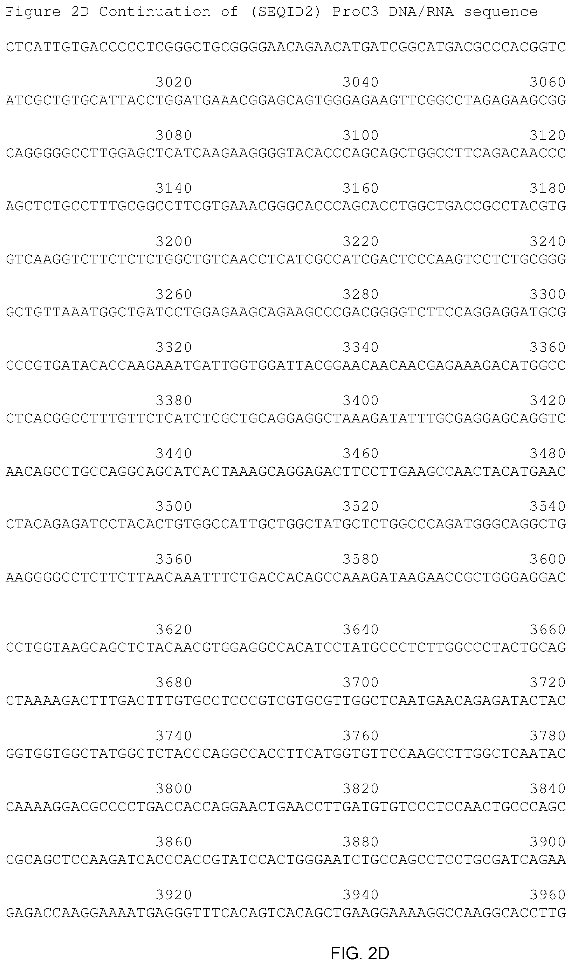

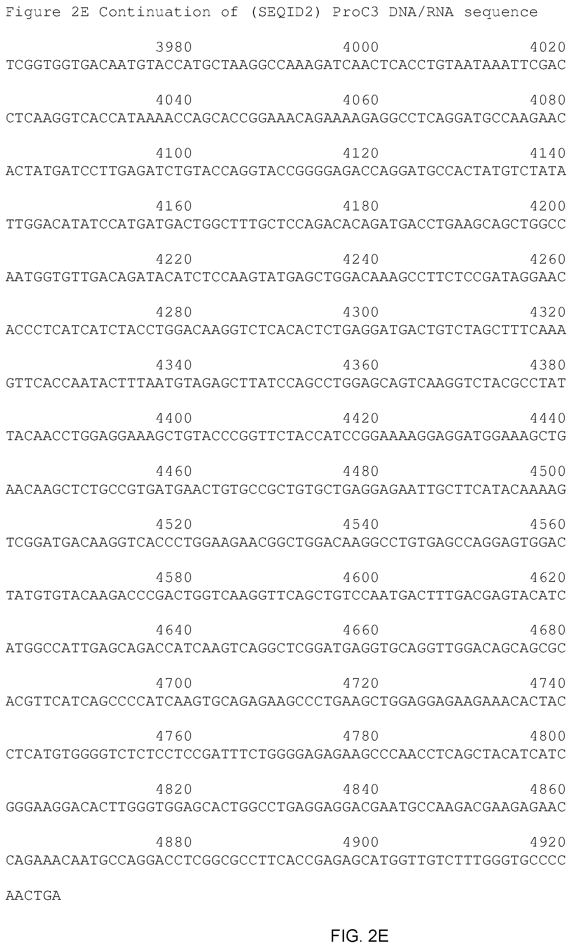

Since the preparation of many/most of the modified C3 proteins will involve producing coding sequences with several amino acid substitutions based on the nucleotide sequence (**Figure 2****,** SEQ ID NO:2) encoding the native human C3 protein (**Figure 1****,** SEQ ID NO:1), the most efficient method to produce these proteins would involve using synthesizing the entire coding sequence of the modified protein with the desired amino acid substitutions and the desired restriction enzyme recognition sequences at the 5' and 3' ends of the coding sequence.

### EXAMPLE 2: Expression of Modified human C3 proteins

Modified C3 proteins will be expressed in either *Drosophila* S2 cells, in Baculovirus-infected Sf9 **(****Figure 3****)** or HighFive™ cells, or, for final production in a mammalian protein expression system, such as in COS7 or CHO cells. For production in *Drosophila* S2 cells, the *Drosophila* BiP signal sequence will be used so that the expressed proteins will be exported to the media. Plasmids containing DNA coding for the modified C3 proteins described above will be cloned into one of the *Drosophila* S2 expression plasmids (pMT/BiP-V5-HisA, ...HisB, or ...HisC). Use of these plasmids allows expression of the proteins with the *Drosophila* BiP signal sequence, thus ensuring high expression and export in S2 cells. pMT/BiP-V5-HisA, B, or C plasmids containing the coding sequence of a modified C3 protein will be transfected into *Drosophila* S2 cells using the calcium phosphate method of Chen and Okayama **(15).** S2 cells were transfected with a mixture of expression plasmid and pCoBlast, using a ratio of 19:1 (w:w). Following transfection, cells containing both plasmids were selected using blasticidin (25 µg/ml). For expression, 1-liter cultures of transfected cells were grown in serum-free medium (High Five™plus L-glutamine), in the absence of blasticidin. When the cells reached a density of 5 x 10⁶cells/ml, production of the recombinant proteins was induced by the addition of CuSO4 to a final concentration of 25 µM. Cultures were allowed to express recombinant proteins for 4-5 days. Hybrid proteins were then purified from the media by a combination of ANX, Sephacryl H-300, and CM-FF chromatography.

For Baculovirus-infected insect cell production of modified C3 proteins, the coding sequences will be cloned into the Baculovirus co-transfection plasmid pBacPAK8. The C3-coding sequence containing plasmid will then be co-transfected into insect cells (Sf9 or HighFive™), using a lipotransfection reagent **(16).** The cells will be allowed to grow for 4-5 days at 27°C. The cell supernatant will be centrifuged to remove cell debris, and approximately 0.5 ml of the P0 virus stock will be used to infect Sf9 or HighFive™ cells growing in log phase. Cells will be removed by centrifugation. This is the P1 virus stock. This stock will be used to infect Sf9 cells at an MOI of 0.1, to create the P2 virus stock, which needs to be filtered through 0.2 micron filters to ensure the removal of all cellular debris. This is repeated to prepare the P3 virus stock, which should have a titer of >5x10⁸pfu/ml. This stock is used for protein expression.

For protein expression in Baculovirus-infected cells, cultures in growing in log phase will be infected the P3 supernatant at an MOI of 3. The culture will be harvested at a time that had been previously determined, and the cells separated from the supernatant by centrifugation.

For expression of modified C3 proteins in mammalian cells, coding sequences for modified C3 proteins will be cloned into pSecTag2A, B, or C, depending on the restriction sites at either end of the modified C3 coding sequence. Since these vectors contain the mouse IgK secretion signal, the coding sequence insert will not contain the human C3 signal sequence **(17).** Coding sequences will be cut with the appropriate restriction enzymes, and ligated into the appropriate pSecTag2/Hygro vector cut with the same enzymes. Following ligation, plasmids will be transformed into *E. coli* DH5αF', and plated out on LB plates containing 100 µg/ml Ampicillin. Plasmids will be isolated, and correct clones determined by restriction mapping and sequencing. Plasmids will be transfected into COS7 cells using the Lipofectamine transfection protocol. Cells containing the Hygromycin B resistance gene will be selected by culturing the cells in media containing varying concentrations of Hygromycin B. Expression of the modified C3 will be determined by PAGE on cell supernatants, and detection using goat antihuman C3 antibodies.

### EXAMPLE 3: Activity Measurements of Modified Human C3 Proteins

The purified modified human C3 proteins will be subjected to a number of functional analyses as follows.

### Complement depletion

This assay measures the ability of a protein to deplete complement in human (or other) serum. The assay was done in two steps. In the first step, the protein of interest will be diluted to the desired concentrations in buffer, usually by serial dilution (typically from less than a nanogram/microliter up to approximately 320 ng/microliter or 3.2 µg in the 10 microliters used in the assay). Then, a 1 µL aliquot of the diluted protein will be mixed with 9 µL undiluted serum. The mixture will be incubated at 37 °C for 3 hours, allowing the protein to exhaustively activate and thus deplete C3 and factor B in the serum. Then, to measure the amount of complement activity left, the serum will be diluted and mixed with antibody-sensitized sheep erythrocytes, which are easily lysed by complement when it is present in serum. This reaction will be allowed to proceed for 30 minutes, and will be stopped by diluting the mixture in cold buffer. The cells will be centrifuged and the lysed cells quantified by measuring the hemoglobin released **(12).**

### Factor B Activation Assay

This is an assay to measure the ability of a modified protein to activate factor B and form a C3/C5 convertase. The convertase formation will be measured as a function of the cleavage of factor B into Bb and Ba. In the assay, purified modified C3 proteins will be incubated with a three-fold molar excess of factor B and catalytic amounts of factor D (all highly purified) in the presence of magnesium at 37°C. At various times, aliquots of the reaction will be withdrawn, and the reaction stopped by adding EDTA, which chelates the magnesium. The reaction products will be run on a non-reducing SDS-polyacrylamide gel, which will be stained for proteins with Coomassie Blue. The amount of Factor B converted will be quantified by scanning the gel into a specialized computer program and measuring the amount of protein in the factor B and Bb bands. The results of this assay are dependent on both the rate of factor B activation and the stability of the resulting convertase. Since there is an excess of factor B in the reaction, a very rapid production of Ba and Bb would indicate an unstable convertase **(12).**

### C3 Convertase Activity Assay

This assay measures the activity of C3/C5 convertases containing modified proteins to activate human C3, by cleaving off the C3a peptide. To perform this assay, convertases will be formed as described above, and the reaction stopped by the addition of EDTA. The convertase will then be mixed with human C3, and the reaction incubated at 37°C. At the indicated times, aliquots will be removed, and the reaction stopped by mixing with gel loading buffer containing SDS and β-mercaptoethanol. The SDS denatures the proteins, and the β-mercaptoethanol reduces the disulfide bonds between cysteines in the proteins. After electrophoresis under reducing conditions, the gel will be stained with Coomassie Blue dye, and the relative amounts of the C3a-chain and the C3a-chain will be quantified as described above. Care is taken to use the same amount of convertase in each reaction. The results of this assay are dependent both on the activity of the modified C3-containing convertase and its stability, as an active but unstable convertase will rapidly cleave C3, but will stop as the convertase dissociates **(12).**

### Assay for cleavage of modified C3 proteins by factors H and I

Modified C3 proteins will incubated with purified human Factor H and Factor I at 37 °C for several hours. The reactions are analyzed by subsequent 7% (w/v) SDS polyacrylamide gel electrophoresis under reducing conditions. Factor H binding and I activity is determined by the reduction in the strength of the 105 kDa α'-chain band, and appearance of bands with a molecular weight of 37 and 40 kDa **(12).**

### Assays for immunogenicity.

Various methods can be used to analyze immunogenicity, including but not limited to, skin tests, testing the modified C3 protein in transgenic animals which have been genetically engineered to have human immune systems, *in vitro* methods, including RIA tests using serum generated in such transgenic animals, radioimmunoprecipitation assays, ELISA assays, electrochemiluminescence, and Surface Plasmon Resonance. In addition, mouse, rat or guinea pig analogs of some proteins can be constructed, using either mouse, rat or guinea pig C3 sequences. These can be injected into the appropriate animal, and serum is collected and analyzed for the production of antibodies against the modified proteins.

This method measures the stability of the modified C3 protein in plasma in different ways. However, it is to be understood that one or all of the methods can be used as well as any other methods known to one of skill in the art.

The first method measures the stability in serum *in vitro.* Rabbit serum is isolated and separated from whole blood. Aliquots of different concentrations of the modified C3 proteins that have been biotinylated are added to the serum and allowed to incubate. Aliquots of the serum are removed at various time intervals, and the amount of modified C3 that persists is identified in an ELISA assay using a monoclonal antibody which is specific to human C3. This is one example of animal serum that can be used. The choice of serum will depend on the cross-reactivity of the human C3 antibodies with C3 of that species.

A second method allows the determination of modified C3 in any animal. Modified C3 proteins are biotinylated, and injected into an animal. At times, blood is withdrawn from the animal, and serum separated from the blood. The amount of biotinylated protein can be measured by ELISA, using biotin antibodies (or streptavidin), and the activity of the modified protein measured by measuring the amount of C3 remaining in the serum, using the second part of the complement depletion assay to determine remaining complement activity.

All illustrations of the drawings are for the purpose of describing selected versions of the present invention and are not intended to limit the scope of the present invention.

## Claims

1. A human C3 protein (SEQ ID NO 1) or an active fragment thereof modified to form a stable C3 convertase, with one amino acid replacement at position 1518 selected from the group consisting of: N, Q, H, S, T, or β-hydroxy-norvaline, wherein the position of the amino acid residue is based on the human Pro-C3 protein numbering.

2. The modified C3 protein according to claim 1 with polyethylene glycol covalently bound to the N-terminus, C-terminus, or a lysine residue on the modified protein, wherein the bound polyethylene glycol increases the half-life and stability of the modified protein in the blood when the modified C3 protein is injected parenterally, and reduces the immunogenicity of the modified protein, and wherein prior to the polyethylene glycol binding reaction, the active site of the modified protein is protected from alteration by pre-incubating with staphylococcal complement inhibitor protein (SCIN).

3. The modified C3 protein according to claim 1-with further sequence changes by adding 1 to 19 amino acids of a non-C3 signal peptide to the N-terminus of the modified protein.

4. The nucleic acid sequence for a modified C3 protein according to claim 1 inserted into a viral vector.

5. A composition comprising the modified C3 protein according to claim 1 and a pharmaceutically acceptable carrier.

6. A nucleic acid sequence encoding the modified human C3 protein according to claim 1.

7. A plasmid or viral vector containing the nucleic acid sequence of claim 6, designed for expression of the modified human C3 protein in a host cell.

8. A modified C3 protein as claimed in claim 1 in an effective amount for use in depleting complement in a patient.

9. A modified C3 protein as claimed in claim 8 for use in local administration into an organ or subcutaneous administration into a cavity or a tissue.

10. A modified C3 protein for use as claimed in claim 9 wherein the administration is a systemic administration, and the systemic administration is intravenous or intraperitoneal.

11. A modified C3 protein as claimed in claim 1 in an effective amount for use in avoiding or ameliorating reperfusion injury in a patient by depleting complement and permitting reperfusion in the patient.

12. A modified C3 protein for use as claimed in claim 11, wherein the modified C3 protein has polyethylene glycol covalently bound to the N-terminus, C-terminus or a lysine residue of the modified protein, and wherein the bound polyethylene glycol increases the half-life and stability of the modified C3 protein in the blood after parenteral injection and decreases immunogenicity.

13. A modified C3 protein as claimed in claim 1 in an effective amount for use in ameliorating effects caused by a disease by depleting complement, wherein the disease is selected from the group consisting of: Neuromyelitis Optica, Multiple Sclerosis, Myasthenia Gravis, Rheumatoid arthritis, wet and dry Age-Related Macular Degeneration, Hemoglobin Urinary Syndrome, Paroxysmal Nocturnal Hemoglobinemia, Inflammatory Bowel Disease, Crohn's Disease, Alzheimer's disease, Parkinson's Disease, Amyotrophic Lateral Sclerosis, Familial Mediterranean Fever, Dengue Fever, Cardiac or any Reperfusion Injury, Gout, Dense Deposit Disease, C3 Glomerulonephritis, Neuropathic Pain, and Inflammatory Pain.

## Patentansprüche

1. Humanes C3-Protein (SEQ ID NO 1) oder ein aktives Fragment davon, modifiziert, um eine stabile C3-Konvertase zu bilden, mit einem Aminosäureaustausch an Position 1518, ausgewählt aus der Gruppe bestehend aus: N, Q, H, S, T oder β-Hydroxynorvalin, wobei die Position des Aminosäurerests auf der Nummerierung des humanen Pro-C3-Proteins basiert.

2. Modifiziertes C3-Protein nach Anspruch 1 mit kovalent an den N-Terminus, C-Terminus oder einen Lysinrest am modifizierten Protein gebundenem Polyethylenglykol, wobei das gebundene Polyethylenglykol die Halbwertszeit und Stabilität des modifizierten Proteins im Blut erhöht, wenn das modifizierte C3-Protein parenteral injiziert wird, und die Immunogenität des modifizierten Proteins verringert, und wobei vor der Polyethylenglykol-Bindungsreaktion die aktive Stelle des modifizierten Proteins durch Vorinkubation mit Staphylokokken-Komplement-Inhibitor-Protein (SCIN) vor Veränderung geschützt ist.

3. Modifiziertes C3-Protein nach Anspruch 1 mit weiteren Sequenzänderungen durch Zugabe von 1 bis 19 Aminosäuren eines Nicht-C3-Signalpeptids zum N-Terminus des modifizierten Proteins.

4. Nukleinsäuresequenz für ein modifiziertes C3-Protein nach Anspruch 1, eingefügt in einen viralen Vektor.

5. Zusammensetzung, umfassend das modifizierte C3-Protein nach Anspruch 1 und einen pharmazeutisch verträglichen Träger.

6. Nukleinsäuresequenz, die für das modifizierte humane C3-Protein nach Anspruch 1 kodiert.

7. Plasmid oder viraler Vektor, enthaltend die Nukleinsäuresequenz nach Anspruch 6 und bestimmt zur Expression des modifizierten humanen C3-Proteins in einer Wirtszelle.

8. Modifiziertes C3-Protein nach Anspruch 1 in einer wirksamen Menge zur Verwendung bei der Komplementdepletion bei einem Patienten.

9. Modifiziertes C3-Protein nach Anspruch 8 zur Verwendung bei der lokalen Verabreichung in ein Organ oder zur subkutanen Verabreichung in einen Hohlraum oder ein Gewebe.

10. Modifiziertes C3-Protein zur Verwendung nach Anspruch 9, wobei die Verabreichung eine systemische Verabreichung ist und die systemische Verabreichung intravenös oder intraperitoneal ist.

11. Modifiziertes C3-Protein nach Anspruch 1 in einer wirksamen Menge zur Verwendung bei der Vermeidung oder Besserung einer Reperfusionsverletzung bei einem Patienten durch Komplementdepletion und Ermöglichen einer Reperfusion bei dem Patienten.

12. Modifiziertes C3-Protein zur Verwendung nach Anspruch 11, wobei bei dem modifizierten C3-Protein Polyethylenglykol kovalent an den N-Terminus, C-Terminus oder einen Lysinrest des modifizierten Proteins gebunden ist und wobei das gebundene Polyethylenglykol die Halbwertzeit und Stabilität des modifizierten C3-Proteins im Blut nach parenteraler Injektion erhöht und Immunogenität verringert.

13. Modifiziertes C3-Protein nach Anspruch 1 in einer wirksamen Menge zur Verwendung bei der Besserung von durch eine Krankheit durch Komplementdepletion verursachten Wirkungen, wobei die Krankheit ausgewählt ist aus der Gruppe bestehend aus:
Neuromyelitis optica, Multipler Sklerose, Myasthenia gravis, rheumatoider Arthritis, feuchter und trockener altersbedingter Makuladegeneration, Hämoglobin-Harn-Syndrom, paroxysmaler nächtlicher Hämoglobinämie, entzündlicher Darmerkrankung, Morbus Crohn, Alzheimer-Krankheit, Parkinson-Krankheit, amyotropher Lateralsklerose, familiärem Mittelmeerfieber, Dengue-Fieber, Herz- oder einer beliebigen Reperfusionsverletzung, Gicht, membranoproliferative Glomerulonephritis Typ II, C3-Glomerulonephritis, neuropathischen Schmerzen und entzündlichen Schmerzen.

## Revendications

1. Protéine C3 humaine (SEQ ID NO 1) ou fragment actif de celle-ci modifiée pour former une convertase C3 stable, avec un remplacement d'acide aminé à la position 1518 choisi dans le groupe constitué de : N, Q, H, S, T ou β-hydroxy-norvaline, dans laquelle la position du résidu d'acide aminé est basée sur la numérotation de protéine Pro-C3 humaine.

2. Protéine C3 modifiée selon la revendication 1 avec du polyéthylèneglycol lié par covalence à l'extrémité N-terminale, à l'extrémité C-terminale ou à un résidu de lysine sur la protéine modifiée, dans laquelle le polyéthylèneglycol lié augmente la demi-vie et la stabilité de la protéine modifiée dans le sang lorsque la protéine C3 modifiée est injectée par voie parentérale, et réduit l'immunogénicité de la protéine modifiée, et dans laquelle, avant la réaction de liaison du polyéthylèneglycol, le site actif de la protéine modifiée est protégé d'une altération par pré-incubation avec une protéine inhibitrice de complément staphylococcique (SCIN).

3. Protéine C3 modifiée selon la revendication 1, avec d'autres changements de séquence par ajout de 1 à 19 acides aminés d'un peptide signal non C3 à l'extrémité N-terminale de la protéine modifiée.

4. Séquence d'acides nucléiques pour une protéine C3 modifiée selon la revendication 1 insérée dans un vecteur viral.

5. Composition comprenant la protéine C3 modifiée selon la revendication 1 et un excipient acceptable sur le plan pharmaceutique.

6. Séquence d'acides nucléiques codant pour la protéine C3 humaine modifiée selon la revendication 1.

7. Plasmide ou vecteur viral contenant la séquence d'acides nucléiques de la revendication 6, conçu pour une expression de la protéine C3 humaine modifiée dans une cellule hôte.

8. Protéine C3 modifiée selon la revendication 1 en une quantité efficace pour utilisation dans une déplétion de complément chez un patient.

9. Protéine C3 modifiée selon la revendication 8 pour utilisation dans une administration locale dans un organe ou une administration sous-cutanée dans une cavité ou un tissu.

10. Protéine C3 modifiée pour utilisation selon la revendication 9, dans laquelle l'administration est une administration systémique et l'administration systémique est intraveineuse ou intrapéritonéale.

11. Protéine C3 modifiée selon la revendication 1 en une quantité efficace pour utilisation dans l'évitement ou l'amélioration d'une lésion de reperfusion chez un patient en réalisant une déplétion de complément et en permettant une reperfusion chez le patient.

12. Protéine C3 modifiée pour utilisation selon la revendication 11, dans laquelle la protéine C3 modifiée a du polyéthylèneglycol lié par covalence à l'extrémité N-terminale, à l'extrémité C-terminale ou à un résidu de lysine de la protéine modifiée, et dans laquelle le polyéthylèneglycol lié augmente la demi-vie et la stabilité de la protéine C3 modifiée dans le sang après une injection parentérale et diminue l'immunogénicité.

13. Protéine C3 modifiée selon la revendication 1 en une quantité efficace pour utilisation dans l'amélioration d'effets provoqués par une maladie par une déplétion de complément, dans laquelle la maladie est choisie dans le groupe constitué de : neuromyélite optique, sclérose en plaques, myasthénie grave, polyarthrite rhumatoïde, dégénérescence maculaire liée à l'âge sèche et humide, syndrome d'hémoglobinurie, hémoglobinurie paroxystique nocturne, maladie intestinale inflammatoire, maladie de Crohn, maladie d'Alzheimer, maladie de Parkinson, sclérose latérale amyotrophique, fièvre méditerranéenne familiale, dengue, lésion cardiaque ou lésion quelconque de reperfusion, goutte, glomérulonéphrite membrano-proliférative, glomérulonéphrite C3, douleur neuropathique et douleur inflammatoire.
